# EUROPEAN PATENT SPECIFICATION

(11) **EP 2 320 894 B1**
(45) Date of publication and mention of the grant of the patent: **28.12.2016**
(21) Application number: 09791732.2
(22) Date of filing: 20.08.2009
(51) Int. Cl.: A61K 31/436, A61P 35/00

(54) **CHEMOPREVENTION OF HEAD AND NECK SQUAMOUS CELL CARCINOMAS**
CHEMOPRÄVENTION VON PLATTENZELLKARZINOMEN AN KOPF UND HALS
CHIMIOPRÉVENTION DE CARCINOME À CELLULES SQUAMEUSES DE LA TÊTE ET DU COU

(30) Priority: 20.08.2008 US 90414 P
(43) Date of publication of application: 18.05.2011
(73) Proprietor: The United States of America, as represented by the Secretary, Department of Health and Human Services, Bethesda, MD 20892-7660 (US)
(72) Inventor: GUTKIND, J. Silvio, Del Mar, CA 92014-3945 (US); AMORNPHIMOLTHAM, Panomwat, Bethesda Maryland 20814 (US); PATEL, Vyomesh, Washington District of Columbia 20009 (US); MOLINOLO, Alfredo, Rockville Maryland 20852 (US); CZERNINSKI, Rakefet, 90805 Measurer-Zion (IL)
(74) Representative: Pfenning, Meinig & Partner mbB
(86) International application number: PCT/US2009/054478
(87) International publication number: WO 2010/022243

(56) References cited:
- WO-A-2004/004644
- WO-A-2008/027013
- NATHAN CHERIE-ANN O ET AL: "Mammalian target of rapamycin inhibitors as possible adjuvant therapy for microscopic residual disease in head and neck squamous cell cancer" CANCER RESEARCH, vol. 67, no. 5, March 2007 (2007-03), pages 2160-2168, XP002555974 ISSN: 0008-5472
- AMORNPHIMOLTHAM PANOMWAT ET AL: "A retroinhibition approach reveals a tumor cell-autonomous response to rapamycin in head and neck cancer" CANCER RESEARCH, vol. 68, no. 4, February 2008 (2008-02), pages 1144-1153, XP002555975 ISSN: 0008-5472
- AMORNPHIMOLTHAM PANOMWAT ET AL: "Mammalian target of rapamycin, a molecular target in squamous cell carcinomas of the head and neck" CANCER RESEARCH, vol. 65, no. 21, November 2005 (2005-11), pages 9953-9961, XP002555976 ISSN: 0008-5472
- CZERNINSKI R C ET AL: "P2.89. Optimizing the 4NQO oral carcinogenesis model for rapamycin chemoprevention" ORAL ONCOLOGY SUPPLEMENT,, vol. 3, no. 1, 1 July 2009 (2009-07-01), pages 189-190, XP026423681 ISSN: 1744-7895 [retrieved on 2009-07-01]
- RAIMONDI ANA R ET AL: "Rapamycin Prevents Early Onset of Tumorigenesis in an Oral-Specific K-ras and p53 Two-Hit Carcinogenesis Model" CANCER RESEARCH, AMERICAN ASSOCIATION FOR CANCER RESEARCH, BALTIMORE, MD., US, vol. 69, no. 10, 1 May 2009 (2009-05-01), pages 4159-4166, XP009125902 ISSN: 0008-5472 [retrieved on 2009-05-12]
- CZEMINSKI RAKEFET ET AL: "Targeting Mammalian Target of Rapamycin by Rapamycin Prevents Tumor Progression in an Oral-Specific Chemical Carcinogenesis Model" CANCER PREVENTION RESEARCH, CN, vol. 2, no. 1, 1 January 2009 (2009-01-01), pages 27-36, XP009125903 ISSN: 1940-6207

## Description

### CROSS-REFERENCE TO A RELATED APPLICATION

This application claims the benefit of United States Provisional Patent Application No. 61/090,414, filed August 20, 2008, the disclosure of which is incorporated by reference.

### BACKGROUND OF THE INVENTION

Head and neck squamous cell carcinomas (HNSCCs), the vast majority of which arise in the oral cavity, represent the sixth most common cancers in the world, with about 500,000 new cases reported annually. This disease results in nearly 11,000 deaths each year in the United States alone. The most prevalent risk factors involved in the development of these highly aggressive malignancies, such as alcohol and tobacco use, betel nut chewing, and infection with the human papillomavirus (HPV) have been long well recognized. However, the five-year survival rate after diagnosis for HNSCC remains low, approximately 50%, which is considerably lower than that for other cancers, such as those of colorectal, cervix and breast origin.

The poor prognosis of HNSCC patients is likely due to the fact that most patients are diagnosed at advanced disease stages, and often fail to respond to available treatment options. Moreover, the effects of the disease are highly disfiguring to the individual. Accordingly, there is a desire to find a method of preventing the development of the disease. Nathan et al. (Cancer Res. 2007, 67: 2160-2168) discloses mTOR inhibitors as possible adjuvant therapy for microscopic residual disease in head and neck squamous cell cancer. Amornphimoltham et al. (Cancer Res. 2008, 68(4): 1144-1153) teaches that a retroinhibition approach reveals a tumor cell-autonomous reponse to rapamycin in head and neck cancer. Amornphimoltham et al. (Cancer Res. 2005, 65(21): 9953-9961) teaches that mTOR is a molecular target in squamous cell carcinomas of the head and neck. WO 2004/004644 A2 discloses a combination of an mTOR and a tyrosine kinase inhibitor for the treatment of neoplasms. WO 2008/027013 A2 discloses a composition comprising a first agent which is an antagonist of mTOR activity such as rapamycin together with a second agent comprising an angiogenesis inhibitor such as an inhibitor of VEGF and use thereof for preventing the growth or proliferation, or both, of a cell or tissue, and that the composition may also be used to treat or prevent cancer, such as hepatocellular carcinoma.

### BRIEF SUMMARY OF THE INVENTION

The invention provides a method for preventing the development of head and neck squamous cell carcinoma (HNSCC) in a mammal at risk for developing such carcinoma.

### BRIEF DESCRIPTION OF THE SEVERAL VIEWS OF THE DRAWING(S)

Fig. 1A depicts a schedule for administering 4-nitroquinoline-1 oxide (4NQO), which is a carcinogen, or water to mice in the 22-week study on the effect of the carcinogen; for Fig. 1A as well as Fig. 1B, 1C, and 2, see Example 2 for details.
Fig. 1B depicts the tumor-free survival curves for control and 4NQO-treated mice.
Fig. 1C depicts the number of tumors per animal each week and the distribution of their corresponding tumor sizes is represented as indicated (n=20). Note that the 4NQO treatment was stopped at week 16, while tumors continued growing after the carcinogen withdrawal. No tumors were observed in control mice.
Fig. 2 depicts the histopathological analysis of 4NQO-induced oral tumoral lesions, specifically the number of lesions per tongue vs. number of weeks.
Fig. 3A depicts a schedule for 4NQO-treated mice in the treatment with rapamycin. For Fig. 3A-E, see Examples 3-4 for details.
Fig. 3B depicts the effect of rapamycin in reducing tumor formation in the tongues of the mice in accordance with an embodiment of the invention.
Fig. 3C depicts a cartoon of the tongues of the control mice and rapamycin treated mice which are depicted in Fig. 3B.
Fig. 3D depicts the number of SCC lesions per tongue of control and rapamycin treated mice depicted in Fig. 3B.
Fig. 3E depicts the surface area of the tumor among the control and rapamycin treated mice depicted in Fig. 3B.

### DETAILED DESCRIPTION OF THE INVENTION

The mTOR is a serine/threonine protein kinase that regulates cell growth, cell proliferation, cell motility, cell survival, protein synthesis, and transcription. mTOR integrates the input from multiple upstream pathways, including insulin, growth factors (such as IGF-1 and IGF-2), and mitogens. mTOR also functions as a sensor of cellular nutrient and energy levels and redox status. The dysregulation of the mTOR pathway is a contributing factor to various human disease processes, especially various types of cancer.

Rapamycin, also known as sirolimus, is a relatively new immunosuppressant drug used to prevent rejection in organ transplantation, and is especially useful in kidney transplants. Sirolimus is a macrolide first discovered as a product of the bacterium *Streptomyces hygroscopicus* in a soil sample from an island called Rapa Nui, better known as Easter Island. Rapamycin inhibits mTOR through association with its intracellular receptor FKBP12. The FKBP12-rapamycin complex binds directly to the FKBP 12-Rapamycin Binding (FRB) domain of mTOR.

HNSCC includes the squamous cell carcinomas of the oral cavity, pharynx and larynx. HNSCC progression involves the sequential acquisition of genetic and epigenetic alterations in genes encoding tumor suppressors and oncogenes, together with the aberrant activity of signaling networks controlling cell proliferation, differentiation, migration, survival, and death. The most frequent genetic alterations in HNSCC include loss of heterozygosity and promoter silencing of the p16 tumor suppressor gene, and inactivating mutations in the *p53* tumor suppressor gene. HNSCCs often overexpress the epidermal growth factor receptor (EGFR) and some of its active variants such as the truncated mutant form EGFR variant III (EGFRvIII), which causes its constitutive activation.

HNSCC lesions harbor activating mutations in the *ras* oncogenes with variable frequency, e.g., from 3-5% in Western countries to around 30-40% in India, and often overexpress cyclin D and exhibit alterations in the NFκB, Stat, Wnt/β-catenin, TGF-β, and PI3K-Akt-mTOR signaling pathways. An aberrant activation of the Akt/mTOR pathway is a frequent event in HNSCC, the vast majority of which occurs in the oral cavity.

The major risk factor for HNSCC is chronic exposure of epithelia to tobacco smoke and alcohol. environmental factors such as wood and cement dusts as well as human papilloma virus type 16 and 18 (HPV) infection have been related to an increased risk of developing HNSCC. Oropharyngeal tumors are often HPV-positive and compose a distinct clinical and pathological entity with less *p53* mutations and better prognosis as compared to HPV negative tumors. Epstein Barr Virus infection is related to nasopharyngeal SCC in South China and oral cavity tumors are frequent in betel chewers.

Both hereditary and environmental factors are implicated in head and neck carcinogenesis and their roles are difficult to separate. Several cancer prone syndromes are associated with a increased risk of head and neck cancer, including Lynch-II, Bloom syndrome, Fanconi anemia, ataxia telangiectasia and Li-Fraumeni syndrome. But genetic susceptibility to HNSCC is more likely to be due to various degrees of DNA maintenance after exposure to tobacco carcinogens. Mutagen sensitivity tests, polymorphism in DNA repair enzymes or in carcinogens metabolizing enzymes supports the role of heredity in HNSCC. GSTM1 and GSTT1 null phenotypes are associated with an increased risk of HNSCC. Concerning XRCC1, the Arg allele (Arg194Trp) and the Gln allele (Arg399Gln) are also linked to an increased risk of oral and pharyngeal cancers. Accordingly, the invention provides a method for preventing the development of HNSCC in a mammal at risk for developing such carcinoma comprising administering an effective amount of at least one mammalian target of rapamycin (mTOR) inhibitor to the mammal. In accordance with an embodiment, the HNSCC is an oral cancer. In another embodiment, the HNSCC is a cancer of the tongue.

In accordance with an embodiment, method of preventing the development of HNSCC comprises halting or slowing the malignant conversion of precancerous lesions or tumors, for example, reducing the number, surface area, or size of precancerous lesions or tumor surface area. Further, in accordance with an embodiment of the invention, the preventing also includes promoting regression of an advanced carcinogen-induced HNSCC.

As discussed, in accordance with an embodiment of the invention, the mammal at risk for HNSCC could be one who has been exposed to a carcinogen or a carcinogen-containing material such as tobacco smoke or those who chew betel nut or one who has a compromised tumor suppressor gene, e.g., *p53, PTEN,* or *p16^{ink4a}* gene.

In accordance with an embodiment, the invention provides an mTOR inhibitor for use in halting or slowing the malignant conversion of precancerous lesions or tumors thereby preventing the development of primary HNSCC, or promoting the regression of an advanced carcinogen-induced HNSCC in a mammal at risk for developing such carcinoma. In accordance with an embodiment, the invention provides the use of a mTOR inhibitor in the preparation of a medicament for use in halting or slowing the malignant conversion of precancerous lesions or tumors thereby preventing development of primary HNSCC, or promoting the regression of an advanced carcinogen-induced HNSCC in a mammal at risk for developing such carcinoma.

In accordance with the invention, any suitable mTOR inhibitor can be administered, for example, the mTOR inhibitor is selected from the group consisting of rapamycin, prodrugs of rapamycin, 40-*O*-(2-hydroxyethyl)-rapamycin, 32-deoxorapamycin, 16-pent-2-ynyloxy-32-deoxorapamycin, 16-pent-2-ynyloxy-32 (*S* or *R*)-dihydro-rapamycin, 16-pent-2- ynyloxy-32 (*S* or *R*)-dihydro-40-*O*-(2-hydroxyethyl)- rapamycin, 40-[3-hydroxy-2-(hydroxy-methyl)-2-methylpropanoate]-rapamycin (CCI779), 40-epi-(tetrazolyl)-rapamycin (ABT578), 42-O-(2-hydroxy)ethyl rapamycin (RAD001), AP23573, TAFA-93, and biolimus, particularly, selected from the group consisting of rapamycin, AP23573, CCI779, RAD001, and TAFA-93.

The term "prodrug" denotes a derivative of a compound, which derivative, when administered to warm-blooded animals, e.g. humans, is converted into the compound (drug). The enzymatic and/or chemical hydrolytic cleavage of the compounds of the present invention occurs in such a manner that the proven drug form (parent carboxylic acid drug) is released, and the moiety or moieties split off remain nontoxic or are metabolized so that nontoxic metabolic products are produced. Non-limiting examples include esters, amides, ethers, and carbonates. Examples of prodrugs of rapamycin include such as those disclosed in United States Patent Application Publication No. 2008/0171763 A1, for example, paragraphs [0029] to [0035], [0037], and [0038], which are incorporated by reference.

In accordance with any of the embodiments, one or more than one, e.g., two, three, or more mTOR inhibitors can be administered.

In accordance with any of the embodiments, one or more mTOR inhibitors can be administered in combination with an additional chemopreventive compound. The additional chemopreventive compound can administered simultaneously or sequentially, i.e., before or after the administration of one or more of mTOR inhibitors. Any suitable chemopreventive compound can be used, for example, one or more selected from the group consisting of vitamin A, retinoids, flavonoids, curcumin analogs, COX-2 inhibitors, ONYX-015, tyrosine kinase inhibitors, angiogenesis inhibitors, selenium, folic acid, polyphenols, statins, metformin, resveratrol, and combinations thereof. Examples of angiogenesis inhibitors include Grb2-SH2 domain binding inhibitors, SU5416, SU6668, cetuximab, gefitinib, erlotinib, canertinib, EKB-569, lapatinib, IMC-C225, ABX-EGF, HuMax-EGFR, DC101, suramin, gleevec, herceptin, p-53 (PRIMA-1), thalidomide, squalamine, anti-αᵥB₃ integrin antibody, anti-αvB5 integrin antibody, cyclic peptide inhibitor of integrin αᵥB₃//αᵥB₅, cilengitide, fumagallin, TNP-470, EMD 121974, α2-antiplasmin, α2-macroglobulin, kininostatin, BMS275291, COL-3, marimastat, neovastat, solimastat, angiostatin, endostatin, antithrombin fragments, fibrinogen-E, fibrin-D, thrombospondin-1, platelet factor-4, low molecular weight heparins, Vioxx, Celebrex, and interferon-α and β.

In accordance with an embodiment, the method comprises administering a pharmaceutical composition comprising a pharmaceutically acceptable carrier and at least one mTOR inhibitor, and optionally one or more additional chemopreventive compound, e.g., one other than the mTOR inhibitor or inhibitors.

The pharmaceutically acceptable excipients described herein, for example, vehicles, adjuvants, carriers or diluents, are well-known to those who are skilled in the art and are readily available to the public. It is preferred that the pharmaceutically acceptable carrier be one that is chemically inert to the active compounds and one that has no detrimental side effects or toxicity under the conditions of use.

The pharmaceutical compositions can be administered orally or parenterally. Thus the compositions can be administered as oral, sublingual, transdermal, subcutaneous, topical, intravenous, intranasal, intraarterial, intramuscular, intratumoral, peritumoral, interperitoneal, intrathecal, rectal, vaginal, or aerosol formulations.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such carriers as are known in the art.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound, or a prodrug, salt, or solvate thereof, can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene-polypropylene copolymers, (d) amphoteric detergents such as, for example, alkyl-beta-aminopropionates, and 2-alkyl-imidazoline quaternary ammonium salts, and (3) mixtures thereof.

The parenteral formulations will typically contain from about 0.5 to about 25% by weight of the inhibitors in solution. Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multidose sealed containers, such as ampoules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described.

The inhibitors may be made into injectable formulations. The requirements for effective pharmaceutical carriers for injectable compositions are well known to those of ordinary skill in the art. See Pharmaceutics and Pharmacy Practice, J. B. Lippincott Co., Philadelphia, Pa., Banker and Chalmers, eds., pages 238-250 (1982), and ASHP Handbook on Injectable Drugs, Toissel, 4th ed., pages 622-630 (1986).

The inhibitors, alone or in combination with other suitable components, can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer.

The dose administered to the mammal, particularly human and other mammals, in accordance with the present invention should be sufficient to effect the desired response. One skilled in the art will recognize that dosage will depend upon a variety of factors, including the age, condition or disease state, predisposition to disease, genetic defect or defects, extent of carcinogen abuse, use of tobacco or other agents such as betel nut, and body weight of the mammal. The size of the dose will also be determined by the route, timing and frequency of administration as well as the existence, nature, and extent of any adverse side-effects that might accompany the administration of a particular inhibitor and the desired effect. It will be appreciated by one of skill in the art that various conditions or disease states may require prolonged treatment involving multiple administrations.

Suitable doses and dosage regimens can be determined by conventional range-finding techniques known to those of ordinary skill in the art. Generally, the preventive treatment is initiated with smaller dosages that are less than the optimum dose of the compound. Thereafter, the dosage is increased by small increments until the optimum effect under the circumstances is reached. The inventive method typically will involve the administration of about 0.01 mg to about 10 mg of one or more of the inhibitors described above per kg body weight of the individual. For example, in embodiments, the inhibitors may be administered in an amount from about 0.05 mg/kg to about 5 mg/kg, from about 0.1 mg/kg to about 3 mg/kg, from about 0.5 mg/kg to about 2.5 mg/kg, from about 1 mg/kg to about 2 mg/kg, from about 1.5 mg/kg to about 1.8 mg/kg, of subject body weight per day, one or more times a day, to obtain the desired therapeutic effect.

By "preventing the development" is meant any degree (10, 20, 30, 40, 50, 60, 70, 80, 90% or more) in inhibition of the onset of cancer or progression of precancerous lesions into cancer, including complete inhibition of development.

Topically applied compositions are generally in the form of liquids, creams, pastes, lotions and gels. Topical administration includes application to the oral mucosa, which includes the oral cavity, oral epithelium, palate, gingival, and the nasal mucosa. In some embodiments, the composition contains at least one active component and a suitable vehicle or carrier. It may also contain other components, such as an anti-irritant. The carrier can be a liquid, solid or semi-solid. In embodiments, the composition is an aqueous solution. Alternatively, the composition can be a dispersion, emulsion, gel, lotion or cream vehicle for the various components. In one embodiment, the primary vehicle is water or a biocompatible solvent that is substantially neutral or that has been rendered substantially neutral. The liquid vehicle can include other materials, such as buffers, alcohols, glycerin, and mineral oils with various emulsifiers or dispersing agents as known in the art to obtain the desired pH, consistency and viscosity. It is possible that the compositions can be produced as solids, such as powders or granules. The solids can be applied directly or dissolved in water or a biocompatible solvent prior to use to form a solution that is substantially neutral or that has been rendered substantially neutral and that can then be applied to the target site. In embodiments of the invention, the vehicle for topical application to the skin can include water, buffered solutions, various alcohols, glycols such as glycerin, lipid materials such as fatty acids, mineral oils, phosphoglycerides, collagen, gelatin and silicone based materials.

In accordance with any of the embodiment, the mTOR inhibitor can be administered topically to premalignant lesions.

The following examples further illustrate the invention but, of course, should not be construed as in any way limiting its scope.

### EXAMPLE 1

This example illustrates the methodology used in carrying out tests in support of embodiments of the invention.

Materials: 4-Nitroquinoline-1 oxide (4NQO) obtained from Sigma Aldrich (St Louis, MO) was dissolved in propylene glycol (Sigma Aldrich) as stock solution (4 mg/ml), stored at 4°C, and diluted in the drinking water to a final concentration of 50 µg/ml. Water was changed weekly. Rapamycin was purchased from LC Laboratories (MA) dissolved in 100% ethanol to a 20x concentration and stored at -80°C. Prior to its intraperitoneal administration (5 mg/kg), this concentrated stock of rapamycin was diluted in an aqueous solution of 5.2% Tween 80 and 5.2% polyethylene glycol (USB, Cleveland, OH and Polysciences Inc, Warrington, PA, respectively).

Methods: Animal studies were carried out according to NIH-approved protocols, in compliance with the Guide for the Care and Use of Laboratory Animals. Female C57B1/6 mice (Harlan Sprague-Dawley, National Cancer Institute at Frederick, MD, USA) 4-6 weeks old and weighing 18 to 20g, were housed in appropriate sterile filter-capped cages, and fed and given water ad libitum. Mice were given either water (control) or 4NQO in the drinking water for 16 weeks, after which all animal cages were reverted to regular water and mice monitored until week 22. All animals underwent a bi-weekly full oral cavity examination under anesthesia (2-5% Isoflurane, Baxter, Deerfield, IL, mixed with oxygen), and any observed pathological changes were documented. Animals were euthanized either on week 16 or week 22 for tissue retrieval. Complete autopsies were performed, and tissues were immediately collected and fixed in buffered zinc-formalin (Z-fix, Anatech, MI) at room temperature for 12 h. The tissues were then transferred to alcohol 70%, processed for paraffin embedding for histopathological diagnosis and further studies. For the analysis of the effect of rapamycin on tumor development, mice were exposed to the carcinogen for 16 weeks, examined, and randomly distributed into treatment and control groups, which received daily intraperitoneal (i.p.) injections with rapamycin (5 mg/kg/day) or an equal volume of diluent (an aqueous solution of 5.2% Tween-80 and 5.2% PEG), respectively. Animals were monitored daily for general behavioral abnormalities, signs of toxicity, illness or discomfort. Animals were euthanized on week 22, and tissue retrieval was performed as described above.

Immunohistochemistry: All antibodies used were from Cell Signaling Technology, Inc., except for anti-COX-2 and anti-bromodeoxyuridine (BrDU) (BD Transduction Laboratories and Accurate Chemical, respectively). The antibodies were used in the concentration as following: p53 rabbit monoclonal antibody, 1:40; phospho-Akt (Ser473) rabbit monoclonal antibody, 1:50; phospho-S6 (pS6) monoclonal rabbit antibody, 1:100; COX-2 mouse monoclonal antibody; 1:100 and BrDU rat monoclonal antibody, 1:10. Tissue slides were dewaxed in Safeclear II, hydrated through graded alcohols and distilled water, and washed three times with PBS. Antigen retrieval was done using an unmasking solution (Vector Laboratories) or 10 mmol/L citric acid boiled in a microwave for 20 min (2 min at 100% power and 18 min at 10% power). The slides were allowed to cool down for 30 min at room temperature, rinsed twice with PBS, and incubated in 3% hydrogen peroxide in PBS for 10 min to quench the endogenous peroxidase. The sections were then sequentially washed in distilled water and PBS and incubated in blocking solution (2.5% bovine serum albumin in PBS) for 30 min at room temperature. Excess solution was discarded, and the sections were incubated with the primary antibody diluted in blocking solution at 4 °C overnight. After washing with PBS, the slides were sequentially incubated with the biotinylated secondary antibody (1:400; Vector Laboratories) for 30 min, and the avidin-biotin complex, reconstituted according to the instruction of the manufacturer in PBS (Vector Stain Elite, ABC kit; Vector Laboratories) for 30 min at room temperature. The slides were developed in 3,3-diaminobenzidine (Sigma FASTDAB tablet; Sigma Chemical) diluted in distilled water, under microscopic control. Development was stopped in distilled water. The slides were thoroughly washed, counterstained with Mayer's hematoxylin, dehydrated, and mounted.

Statistical analysis: ANOVA followed by Bonferroni's multiple comparison tests were used to analyze the differences of tumor mass volume between experimental groups. Mann Whitney test was used to evaluate differences in total surfaces and tumor multiplicity. Data analysis was performed with using GraphPad Prism version 4.00 for Windows (GraphPad Software La Jolla, CA); P values <0.05 were considered statistically significant.

### EXAMPLE 2

This example illustrates an experimental oral chemical carcinogenesis model to identify HNSCC development and progression in a mammal.

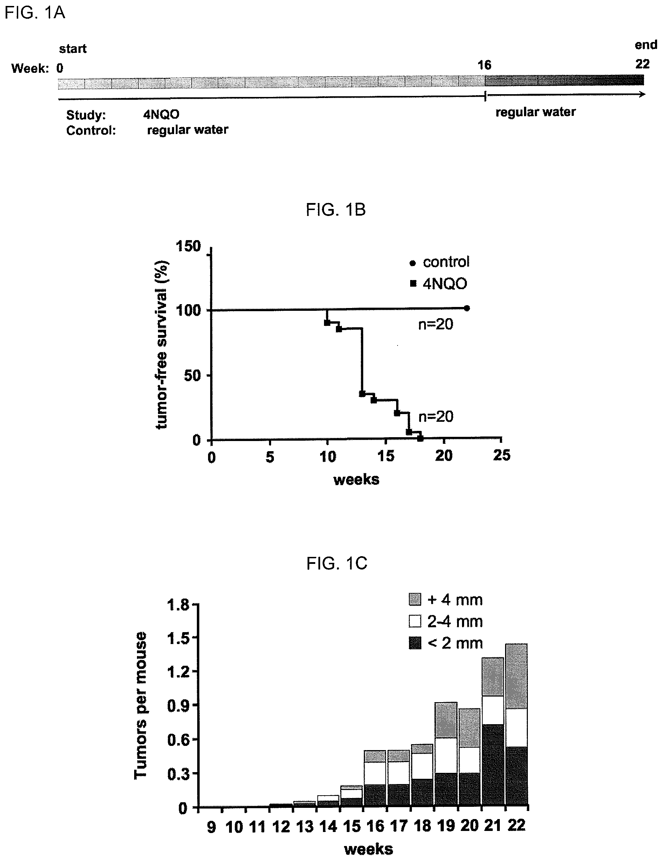

In particular, this example illustrates the use of 4-nitroquinoline-1 oxide (4NQO), a synthetic water soluble organic compound that forms DNA-adducts thereby causing adenosine for guanosine substitutions and induces intracellular oxidative stress resulting in mutations and DNA strand breaks, as a carcinogen. The changes in the cellular DNA induced by 4NQO administration are typical from those provoked by tobacco carcinogens, hence serving as a surrogate of tobacco exposure. The extensive optimization of the experimental conditions using a variety of mouse strains led to establish a general procedure for the administration of 4NQO depicted in Fig. 1A, which resulted in the progressive appearance of tumoral lesions in the tongue and oral mucosa that were preceded by clearly identifiable preneoplastic events.

C57B1/6 mice, one of the most frequently utilized mouse strains, were provided water (control) or 4NQO (50 µg/ml) in the drinking water for 16 weeks, a procedure that required minimal intervention. Water was replaced weekly, and mice were monitored prior to each water change. After 16 weeks, the administration of the carcinogen was suspended, and all mice received regular water. All animals underwent a bi-weekly full oral cavity examination, and any pathological changes were documented.

None of the control animals developed any lesions through the end of the study. However, around 50% of the mice exposed to 4NQO developed tumors in the tongue and other areas of the oral mucosa as early as week 12 (Fig. 1B). The lesions were usually papillomatous-like and could be distinguished from the adjacent mucosa by its irregular growth and whiter appearance. At week 16, at the end of the carcinogen exposure, most mice presented visible oral tumoral lesions. The number and size of the tumors continued growing progressively, even after switching to regular water without 4NQO (Fig. 1C). Indeed, most mice developed remarkable tumoral lesions in the tongue, and we also observed discolorations and hypertrophic growth in the palate as well as in the floor of the mouth, some of which evolved into full papillary lesions. Overall, at the end of the study on week 22, all mice exposed to 4NQO developed one or more large oral cavity tumors in repeated experimental groups, supporting the usefulness of this chemical carcinogenesis model to investigate oral cancer development.

Of interest, the highest incidence of tumors in this animal model occurred in the tongue, a location that represents 20-40% of all oral cancers in humans. A detailed histological analysis of the resulting cancerous lesions was conducted in the entire tongue from each animal at the end of the study (week 22). After fixing, each tongue was cut into four sections of approximately the same thickness, following its major axis. These sections were embedded in a single paraffin block and multiple sections were cut and stained with Hematoxylin and Eosin (H&E) for diagnostic purposes and for immunohistochemical analysis. The presence of multiple dysplastic and cancerous lesions was noted. Besides readily visible tumors, there were also a large number of clearly identifiable precancerous lesions in each tongue, which were classified into low grade and high grade, following classical tissue architectural and cytological features.

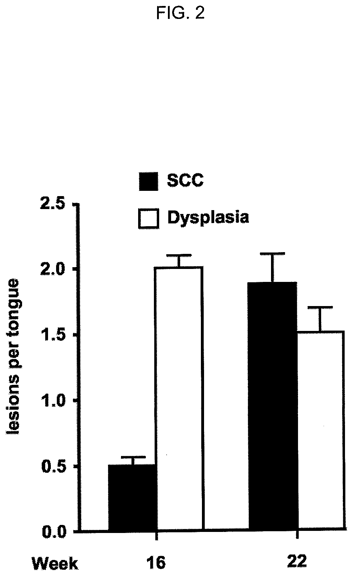

While all papillary lesions were squamous carcinomas, all these preneoplastic lesions were flat, resembling human oral lesions in which premalignant lesions rarely exhibit a papillary aspect, with the exception of proliferative verrucous leukoplakias. Microscopically, all malignant tumors examined were identified as SCCs with different degrees of keratinization. There was an absence of significant inflammatory infiltration, with inflammation becoming evident only in grossly papillary lesions. All dysplasias, microcarcinomas and SCCs were quantified. As shown in Fig. 2, on week 16, at the end of the carcinogen treatment, it was found that every mouse had one or multiple dysplastic lesions, with overall four times fewer carcinomas than dysplasias per tongue. However, at week 22, all mice had one or more large SCC in their tongue, and nearly the same number of dysplastic than SCC lesions. This suggests that 4NQO may provoke the accumulation of genetic changes in the oral epithelial cells, which leads to few malignant but numerous premalignant lesions at the end of the carcinogen exposure, but that many of these lesions may then evolve spontaneously into large carcinomas during the follow up period after carcinogen withdrawal.

The proliferative status of these dysplastic and tumoral lesions was investigated. There were very few isolated BrDU positive nuclei in the tongue mucosa in control animals, but there was an increased BrDU incorporation in proliferating epithelial cells even in the non-neoplastic areas of the tongue in 4NQO-treated mice. In every case, however, the proliferating cells were confined to the basal layer. Aberrant proliferation was evident instead in dysplastic tissues, with cells incorporating BrDU in focal areas, some of which involved suprabasal cells. All SCCs displayed a massive growth of tumoral cells, which included both basal and suprabasal regions. The level of COX-2 expression was evaluated to assess the possible contribution of pro-inflammatory pathways in tumor progression in this chemical carcinogenesis model. COX-2 expression was absent in normal as well as in hyperplastic 4NQO tissues, a feature that was associated with the lack of stromal inflammatory infiltration. Increased expression of COX-2 was detectable in the early dysplastic lesions, and this was even more remarkable in SCCs. However, in both cases, COX-2 expression was restricted to the epithelial cells, suggesting that in this model, cell inflammation involves intrinsic mechanisms triggered in tumoral cells, and not as part of a secondary inflammatory response provoked by the stroma.

Remarkable changes also occurred in the immunodetection of p53, which often reflects the presence of mutations in the p53 tumor suppressor gene, a highly prevalent alteration in HNSCC. Immunoreactive p53 was absent from normal tongue tissues, but isolated foci of p53 positive cells were evident in non-malignant epithelium of 4NQO treated mice, particularly in areas of hyperplasia, reflecting an early compromise of this tumor suppressive protein in the development of tongue SCC. The number of immunoreactive cells increased in precancerous lesions, and was maximal in SCC, with most cells expressing p53 located in the basal and parabasal layers. A progressive dysregulation of the Akt-mTOR pathway was also evident in this carcinogenesis model, as judged by the fact that as the lesions progress to malignancy there was an increase in the number of positive cells for the phosphorylated species of Akt, pAktS473, and S6, pS6, the latter representing one of the most downstream targets of mTOR. An interesting finding was the observation that most cells positive for pS6 and pAktS473 in normal and dysplastic lesions involved parabasal cells, which most likely represent non-proliferating cells undergoing differentiation. In contrast, SCC cells displayed elevated pS6 and pAktS473 in multifocal areas throughout the tumor.

### EXAMPLE 3

This Example illustrates a method of inhibiting the mTOR function with rapamycin in accordance with an embodiment of the invention.

The treatment with rapamycin reduces the tumor burden in the 4NQO-induced oral carcinogenesis model. Mice were given 4NQO (50 µg/ml) in the drinking water for 16 weeks, and then divided into a rapamycin-treated group (5 mg/kg/day; i.p.) and a control group (vehicle control; i.p.), and reverted to regular water until week 22. All animals were euthanized on week 22, and complete autopsies performed. B. Pictures of representative tongues of control and rapamycin-treated mice, showing a decreased tumor burden in the rapamycin treated group. Histological analysis of each tongue enabled the digital representation of each of the SCC lesions in the dorsal tongue. Multiple tissue sections from each tongue were examined, and the number of HNSCC lesions, including large HNSCCs and microcarcinomas, per tongue were represented individually for both, the control and rapamycin treated groups. There was a significant reduction in the number of SCC lesions upon rapamycin treatment. The compromised areas in each tongue were digitally quantified, and the average ± S.E.M. of the surface of the affected area per tongue was represented for the control and rapamycin treated group. A highly significant reduction in the overall size of the oral HNSCC lesions caused by 4NQO was observed in the rapamycin treated group.

Representative tissue sections from the tongue of control mice and non-neoplastic, dysplastic, and cancerous (SCC) regions of mice exposed for 16 weeks to 4NQO and euthanized on week 22, were analyzed for their proliferative status (BrDU), expression of COX-2, and the accumulation of immunoreactive forms of p53 and the phosphorylated species of S6 (pS6) and Akt (pAktS473), as indicated. An increased number of proliferating cells was noted, as well as the distinct pattern and increased levels of COX-2, p53, pS6, and pAktS473, as the tumors progressed.

Administration of rapamycin decreased the activity of mTOR in 4NQO-induced cancerous lesions. The treatment for three days with rapamycin (5 mg/kg/day; i.p.) caused a remarkable reduction in the levels of both pAktS473 and pS6. While few cells remained positive for pS6 and pAktS473 after the rapamycin treatment, they were limited to the upper, most differentiated layers, but absent from the proliferative basal layers, as compared to their high levels in the basal layers of the control, vehicle treated 4NQO-induced SCCs.

Animals exhibiting large SCC tumors at week 22 were treated with rapamycin for 3 consecutive days, which resulted in a dramatic decrease of the levels of pS6 and pAktS473, both targets of mTOR. While pS6 is a downstream target of mTOR complex 1 (mTORC1), which is blocked by rapamycin, the phosphorylation of Akt in its serine 473 represents a target of mTOR complex 2 (mTORC2), which is indirectly inhibited upon prolonged exposure to rapamycin. In both cases, only a limited residual immunodetectable pS6 and pAktS473 was observed in the most differentiated areas of the tumor, suggesting that mTOR may be more resistant to inhibition in this cell population, while the less differentiated and proliferating cells are highly sensitive to this novel anti-neoplastic agent. However, overall, it was confirmed that rapamycin treatment blocked the elevated mTOR activity in the proliferative cell compartment of these oral cancerous lesions.

### EXAMPLE 4

This Example illustrates a method of preventing the development of HNSCC in accordance with an embodiment of the invention.

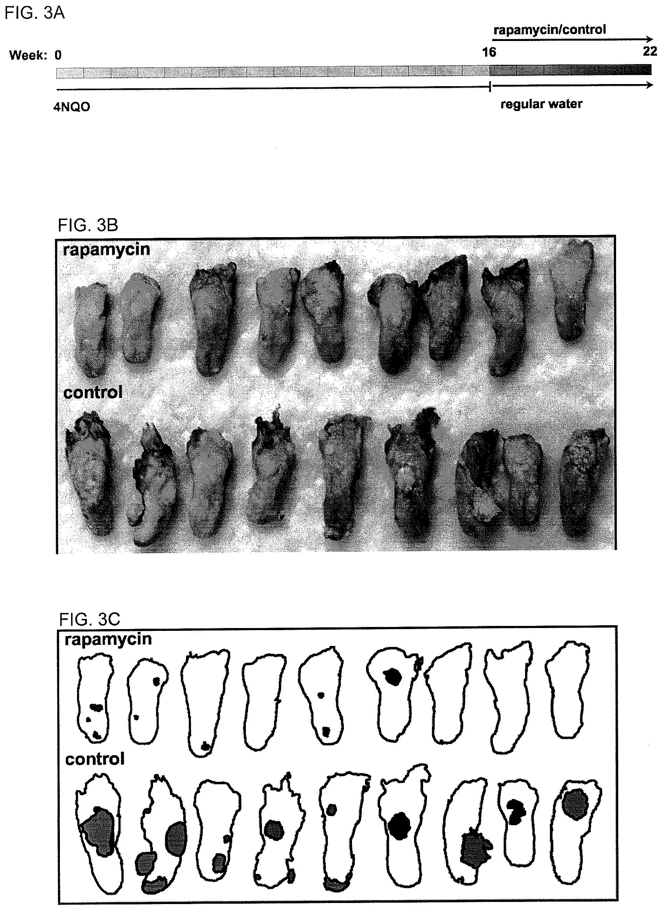

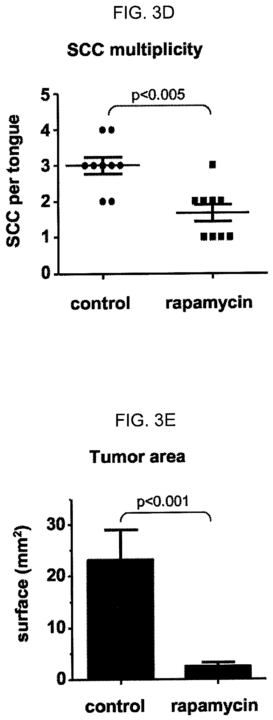

Specifically, rapamycin interferes with tumor development when its administration was initiated at the end of the 4NQO exposure (week 16), thus avoiding any possible interactions between 4NQO and rapamycin. Indeed, following a treatment scheme depicted in Fig. 3A, it was observed that the effects of rapamycin were quite remarkable. At gross examination, differences between the rapamycin treated and control groups were readily evident in both the size and number of tumoral lesions (Fig. 3B). Multiple sections from each tongue were systematically examined as described above, and the number and size of each SCC lesion quantitated and used to reconstruct a topographic map of compromised surfaces in the rapamycin treated and control groups. Cartoons for each tongue depicted the shape of their actual pictures, with the red areas indicating the presence of SCCs, as judged by their histological examination (Fig. 3C). A highly significant difference was observed in the number of SCCs in each tongue between the rapamycin-treated and control groups. When the compromised areas in each tongue were digitally quantified, it was found that rapamycin dramatically diminished both the tumor multiplicity and the overall affected tumor area (Fig. 3D and 3E, respectively). The foregoing shows the effectiveness of blocking mTOR in preventing the development of oral SCC lesions once initiated by chemical carcinogens.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An inhibitor of a mammalian target of rapamycin (mTOR) for use in halting or slowing the malignant conversion of precancerous lesions or tumors thereby preventing development of head and neck squamous cell carcinoma (HNSCC), or promoting the regression of an advanced carcinogen-induced HNSCC, in a mammal at risk for developing such carcinoma.

2. The inhibitor of claim 1 for use according to claim 1, wherein the HNSCC is an oral cancer or a cancer of the tongue.

3. The inhibitor of claim 1, for use in halting or slowing the malignant conversion of precancerous lesions.

4. The inhibitor of claim 3 for use in reducing the number or size of lesions or tumor surface area.

5. The inhibitor of claim 1, for use in promoting the regression of an advanced carcinogen-induced HNSCC.

6. The inhibitor of claim 1 for use according to claim 1, wherein the mammal at risk for HNSCC has been exposed to a carcinogen.

7. The inhibitor of claim 1 for use according to claim 1, wherein the mammal at risk has a compromised tumor suppressor gene, wherein the tumor suppressor gene is selected from the group consisting of *p53*, *PTEN,* and *p16^{inkaα}.*

8. The inhibitor of any one of claims 1 to 7 for use according to any one of claims 1 to 7, which is present in a topical formulation applicable to premalignant lesions.

9. The inhibitor of any one of claims 1 to 7 for use according to any one of claims 1 to 7, which is present in a formulation suitable for administering orally, parenterally, sublingually, transdermally, subcutaneously, topically, intravenously, intranasally, intraarterially, intramuscularly, intratumorally, peritumorally, interperitoneally, intrathecally, rectally, vaginally, or nasally.

10. The inhibitor of any one of claims 1 to 9 for use according to any one of claims 1 to 9, wherein the mTOR inhibitor is selected from the group consisting of rapamycin, esters, ethers, amides, and carbonates of rapamycin, 40-*O*-(2-hydroxyethyl)-rapamycin, 32-deoxorapamycin, 16-pent-2-ynyloxy-32-deoxorapamycin, 16-pent-2-ynyloxy-32 (*S* or *R*)-dihydro-rapamycin, 16-pent-2-ynyloxy-32 (*S* or *R*)-dihydro-40-*O*-(2-hydroxyethyl)- rapamycin, 40-[3-hydroxy-2-(hydroxy-methyl)-2-methylpropanoate]-rapamycin (CCI779), 40-epi-(tetrazolyl)-rapamycin (ABT578), 42-O-(2-hydroxy)ethyl rapamycin (RAD001), AP23573, TAFA-93, and biolimus.

11. The inhibitor of claim 10 for use according to claim 10, wherein the mTOR inhibitor is selected from the group consisting of rapamycin, AP23573, RAD001, CCI779, and TAFA-93.

12. The inhibitor of any one of claims 1 to 11 for use according to any one of claims 1 to 11, wherein the inhibitor is used in combination with one or more additional chemopreventive compounds.

13. The inhibitor of claim 12 for use according to claim 12, wherein the additional chemopreventive compound or compounds is selected from the group consisting of vitamin A, retinoids, flavonoids, curcumin analogs, COX-2 inhibitors, ONYX-015, tyrosine kinase inhibitors, angiogenesis inhibitors, selenium, folic acid, polyphenols, statins, metformin, resveratrol, and combinations thereof.

14. The inhibitor of claim 13 for use according to claim 13, wherein the angiogenesis inhibitor is selected from the group consisting of Grb2-SH2 domain binding inhibitors, SU5416, SU6668, cetuximab, gefitinib, erlotinib, canertinib, EKB-569, lapatinib, IMC-C225, ABX-EGF, HuMax-EGFR, DC101, suramin, gleevec, herceptin, p-53 (PRIMA-1), thalidomide, squalamine, anti-αᵥB₃ integrin antibody, anti-αvB5 integrin antibody, cyclic peptide inhibitor of integrin αᵥB₃//αᵥB₅, cilengitide, fumagallin, TNP-470, EMD 121974, α2-antiplasmin, α2-macroglobulin, kininostatin, BMS275291, COL-3, marimastat, neovastat, solimastat, angiostatin, endostatin, antithrombin fragments, fibrinogen-E, fibrin-D, thrombospondin-1, platelet factor-4, low molecular weight heparins, Vioxx, Celebrex, and interferon-α and β, and any combination thereof.

15. The inhibitor of any one of claims 1 to 9 for use according to any one of claims 1 to 9, wherein the mTOR inhibitor is selected from the group consisting of rapamycin, esters, ethers, amides, and carbonates of rapamycin, 40-*O*-(2-hydroxyethyl)-rapamycin. 32-deoxorapamycin, 16-pent-2-ynyloxy-32-deoxoiapamycin, 16-pent-2-ynyloxy-32 (*S* or *R*)-dihydro-rapamycin, 6-pent-2-ynyloxy-32 (*S* or *R*)-dihydro-40-0-(2-hydroxyethyl)-rapamycin, 40-epi-(tetrazolyl)-rapamycin (ABT578), 42-O-(2-hydroxy)ethyl rapamycin (RAD001), AP23573, TAFA-93, and biolimus.

16. The inhibitor of claim 1 for use according to claim 1, wherein the mammal at risk has a compromised tumor suppressor genre, wherein the tumor suppressor gene is *p53* or *p16^{ink4α}.*

## Patentansprüche

1. Ein Inhibitor eines Targets von Rapamycin in Säugetieren (mTOR) zur Verwendung in der
Blockierung oder Verlangsamung der bösartigen Umwandlung von präkanzerösen Läsionen oder Tumoren, wobei dadurch die Entwicklung von einem Plattenepithelkarzinom am Kopf und Hals (HNSCC) verhindert wird, oder
Förderung der Regression von einem fortgeschrittenen, Karzinogenverursachten HNSCC in einem Säugetier, das gefährdet ist, ein solches Karzinom zu entwickeln.

2. Der Inhibitor von Anspruch 1 zur Verwendung gemäß Anspruch 1, wobei das HNSCC ein Mundkrebs oder ein Zungenkrebs ist.

3. Der Inhibitor von Anspruch 1 zur Verwendung in der Blockierung oder Verlangsamung der bösartigen Umwandlung von präkanzerösen Läsionen.

4. Der Inhibitor von Anspruch 3 zur Verwendung in der Verringerung der Anzahl oder Größe von Läsionen oder der Tumoroberfläche.

5. Der Inhibitor von Anspruch 1 zur Verwendung in der Förderung der Regression von einem fortgeschrittenen, Karzinogen-verursachten HNSCC.

6. Der Inhibitor von Anspruch 1 zur Verwendung gemäß Anspruch 1, wobei das Säugetier mit Risiko für HNSCC einem Karzinogen ausgesetzt war.

7. Der Inhibitor von Anspruch 1 zur Verwendung gemäß Anspruch 1, wobei das gefährdete Säugetier ein beeinträchtigtes Tumorsuppressor-Gen hat, wobei das Tumorsuppressor-Gen ausgewählt ist aus der Gruppe bestehend aus *p53, PTEN und P16^{ink4α}.*

8. Der Inhibitor von einem der Ansprüche 1 bis 7 zur Verwendung gemäß einem der Ansprüche 1 bis 7, wobei der Inhibitor in einer topischen Formulierung, die auf prämaligne Läsionen anwendbar ist, vorhanden ist.

9. Der Inhibitor von einem der Ansprüche 1 bis 7 zur Verwendung gemäß einem der Ansprüche 1 bis 7, wobei der Inhibitor in einer Formulierung vorhanden ist, die zur oralen, parenteralen, sublingualen, transdermalen, subkutanen, topischen, intravenösen, intranasalen, intraarteriellen, intramuskulären, intratumoralen, peritumoralen, interperitonealen, intrathekalen, rektalen, vaginalen, oder nasalen Verabreichung geeignet ist.

10. Der Inhibitor von einem der Ansprüche 1 bis 9 zur Verwendung gemäß einem der Ansprüche 1 bis 9, wobei der mTOR-Inhibitor ausgewählt ist aus der Gruppe bestehend aus Rapamycin, Estern, Ethern, Amiden, und Carbonates von Rapamycin, 40-*O*-(2-Hydroxyethyl)-rapamycin, 32-Deoxorapamycin, 16-Pent-2-inyloxy-32-deoxorapamycin, 16-Pent-2-inyloxy-32-(*S* oder *R*)-dihydrorapamycin, 16-Pent-2-inyloxy-32-(*S* oder *R*)-dihydro-40-*O*-(2-hydroxyethyl)-rapamycin, 40-[3-Hydroxy-2-(hydroxy-methyl)-2-methylpropanoat]-rapamycin (CCI779), 40-Epi(tetrazolyl)-rapamycin (ABT578), 42-O-(2-Hydroxy)ethyl-rapamycin (RAD001), AP23573, TAFA-93, und Biolimus.

11. Der Inhibitor von Anspruch 10 zur Verwendung gemäß Anspruch 10, wobei der mTOR-Inhibitor ausgewählt ist aus der Gruppe bestehend aus Rapamycin, AP23573, RAD001, CCI779, und TAFA-93.

12. Der Inhibitor von einem der Ansprüche 1 bis 11 zur Verwendung gemäß einem der Ansprüche 1 bis 11, wobei der Inhibitor in Kombination mit einem oder mehreren zusätzlichen chemopräventiven Verbindungen verwendet wird.

13. Der Inhibitor von Anspruch 12 zur Verwendung gemäß Anspruch 12, wobei die zusätzliche chemopräventive Verbindung ausgewählt ist oder die zusätzlichen chemopräventiven Verbindungen ausgewählt sind aus der Gruppe bestehend aus Vitamin A, Retinoide, Flavonoide, Kurkumin-Analoga, COX-2-Inhibitoren, ONYX-015, Tyrosinkinaseinhibitoren, Angiogeneseinhibitoren, Selen, Folsäure, Polyphenole, Statine, Metformin, Resveratrol, und Kombinationen hiervon.

14. Der Inhibitor von Anspruch 13 zur Verwendung gemäß Anspruch 13, wobei der Angiogeneseinhibitor ausgewählt ist aus der Gruppe bestehend aus Grb2-SH2-Domäne-bindende Inhibitoren, SU5416, SU6668, Cetuximab, Gefitinib, Erlotinib, Canertinib, EKB-569, Lapatinib, IMC-C225, ABX-EGF, HuMax-EGFR, DC101, Suramin, Gleevec, Herceptin, p-53 (PRIMA-1), Thalidomid, Squalamin, Anti-aᵥB₃ Integrinantikörper, Anti-aᵥB₅ Integrinantikörper, zyklischer Peptidinhibitor von Integrin aᵥB₃//aᵥB₅, Cilengitid, Fumagallin, TNP-470, EMD 121974, α2-Antiplasmin, α,2-Macroglobulin, Kininostatin, BMS275291, COL-3, Marimastat, Neovastat, Solimastat, Angiostatin, Endostatin, Antithrombinfragmenten, Fibrinogen-E, Fibrin-D, Thrombospondin-1, Plättchenfaktor-4, niedermolekulare Heparine, Vioxx, Celebrex, und Interferon-α, und β, und jede Kombination hiervon.

15. Der Inhibitor von einem der Ansprüche 1 bis 9 zur Verwendung gemäß einem der Ansprüche 1 bis 9, wobei der mTOR-Inhibitor ausgewählt ist aus der Gruppe bestehend aus Rapamycin, Estern, Ethern, Amiden, und Carbonates von Rapamycin, 40-*O*-(2-Hydroxyethyl)-rapamycin, 32-Deoxorapamycin, 16-Pent-2-inyloxy-32-deoxorapamycin, 16-Pent-2-inyloxy-32-(*S* oder *R*)-dihydrorapamycin, 16-Pent-2-inyloxy-32-(*S* oder *R*)-dihydro-40-*O*-(2-hydroxyethyl)-rapamycin, 40-Epi(tetrazolyl)-rapamycin (ABT578), 42-*O*-(2-Hydroxy)ethyl-rapamycin (RAD001), AP23573, TAFA-93, und Biolimus.

16. Der Inhibitor von Anspruch 1 zur Verwendung gemäß Anspruch 1, wobei das gefährdete Säugetier ein beeinträchtigtes Tumorsuppressor-Gen hat, wobei das Tumorsuppressor-Gen *p53* oder *P16^{ink4α}* ist.

## Revendications

1. Inhibiteur d'une cible de mammifère de rapamycine (mTOR) pour utilisation dans l'arrêt ou le ralentissement de la conversion maligne de lésions précancéreuses ou de tumeurs de manière à prévenir le développement d'un carcinome à cellules squameuses de la tête et du cou (HNSCC), ou l'induction de la régression d'un HNSCC induit par une substance cancérigène avancé chez un mammifère à risque de développer un tel carcinome.

2. Inhibiteur de la revendication 1 pour utilisation selon la revendication 1, le HNSCC étant un cancer buccal ou un cancer de la langue.

3. Inhibiteur de la revendication 1, pour utilisation dans l'arrêt ou le ralentissement de la conversion maligne de lésions précancéreuses.

4. Inhibiteur de la revendication 3 pour utilisation dans la réduction du nombre ou de la taille de lésions ou la surface de tumeur.

5. Inhibiteur de la revendication 1, pour utilisation dans la promotion de la régression d'un HNSCC induit par une substance cancérigène avancé.

6. Inhibiteur de la revendication 1 pour utilisation selon la revendication 1, le mammifère à risque de HNSCC ayant été exposé à une substance cancérigène.

7. Inhibiteur de la revendication 1 pour utilisation selon la revendication 1, le mammifère à risque ayant un gène suppresseur de tumeur compromis, le gène suppresseur de tumeur étant choisi dans le groupe constitué de *p53, PTEN* et *p16^{ink4a}.*

8. Inhibiteur de l'une quelconque des revendications 1 à 7 pour utilisation selon l'une quelconque des revendications 1 à 7, qui est présent dans une formulation topique applicable à des lésions prémalignes.

9. Inhibiteur de l'une quelconque des revendications 1 à 7 pour utilisation selon l'une quelconque des revendications 1 à 7, qui est présent dans une formulation adaptée pour administration par voie orale, parentérale, sublinguale, transdermique, sous-cutanée, topique, intraveineuse, intranasale, intra-artérielle, intramusculaire, intratumorale, péritumorale, interpéritonéale, intrathécale, rectale, vaginale, ou nasale.

10. Inhibiteur de l'une quelconque des revendications 1 à 9 pour utilisation selon l'une quelconque des revendications 1 à 9, l'inhibiteur de mTOR étant choisi dans le groupe constitué des rapamycine, esters, éthers, amides et carbonates de rapamycine, 40-*O*-(2-hydroxyéthyl)-rapamycine, 32-désoxorapamycine, 16-pent-2-ynyloxy-32-désoxorapamycine, 16-pent-2-ynyloxy-32(*S* ou *R*)-dihydro-rapamycine, 16-pent-2-ynyloxy-32(*S* ou *R*)-dihydro-40-*O*-(2-hydroxyéthyl)-rapamycine, 40-[3-hydroxy-2-(hydroxy-méthyl)-2-méthylpropanoate]-rapamycine (CCI779), 40-épi-(tétrazolyl)-rapamycine (ABT578), 42-O-(2-hydroxy)éthyl-rapamycine (RAD001), AP23573, TAFA-93, et biolimus.

11. Inhibiteur de la revendication 10 pour utilisation selon la revendication 10, l'inhibiteur de mTOR étant choisi dans le groupe constitué des rapamycine, AP23573, RAD001, CCI779, et TAFA-93.

12. Inhibiteur de l'une quelconque des revendications 1 à 11 pour utilisation selon l'une quelconque des revendications 1 à 11, l'inhibiteur étant utilisé en combinaison avec un ou plusieurs composés chimiopréventifs additionnels.

13. Inhibiteur de la revendication 12 pour utilisation selon la revendication 12, le composé ou les composés chimiopréventif(s) additionnel(s) étant choisi(s) dans le groupe constitué des vitamine A, rétinoïdes, flavonoïdes, analogues de curcumine, inhibiteurs de COX-2, ONYX-015, inhibiteurs de tyrosine kinase, inhibiteurs d'angiogenèse, sélénium, acide folique, polyphénols, statines, metformine, resvératrol, et des combinaisons de ceux-ci.

14. Inhibiteur de la revendication 13 pour utilisation selon la revendication 13, l'inhibiteur d'angiogenèse étant choisi dans le groupe constitué d'inhibiteurs de liaison de domaine Grb2-SH2, SU5416, SU6668, cétuximab, géfitinib, erlotinib, canertinib, EKB-569, lapatinib, IMC-C225, ABX-EGF, HuMax-EGFR, DC101, suramine, Gleevec, Herceptin, p-53 (PRIMA-1), thalidomide, squalamine, anticorps anti-intégrine αᵥB₃, anticorps anti-intégrine αᵥB₅, inhibiteur peptidique cyclique d'intégrine αᵥB₃//αᵥB₅, cilengitide, fumagalline, TNP-470, EMD 121974, α2-antiplasmine, α2-macroglobuline, kininostatine, BMS275291, COL-3, marimastat, néovastat, solimastat, angiostatine, endostatine, fragments d'antithrombine, fibrinogène E, fibrine D, thrombospondine 1, facteur plaquettaire 4, héparines de poids moléculaire faible, Vioxx, Celebrex et interféron α et β, et une combinaison quelconque de ceux-ci.

15. Inhibiteur de l'une quelconque des revendications 1 à 9 pour utilisation selon l'une quelconque des revendications 1 à 9, l'inhibiteur de mTOR étant choisi dans le groupe constitué des rapamycine, esters, éthers, amides et carbonates de rapamycine, 40-*O*-(2-hydroxyéthyl)-rapamycine, 32-désoxorapamycine, 16-pent-2-ynyloxy-32-désoxorapamycine, 16-pent-2-ynyloxy-32(*S* ou *R*)-dihydro-rapamycine, 16-pent-2-ynyloxy-32 (*S* ou *R*)-dihydro-40-*O*-(2-hydroxyéthyl)-rapamycine, 40-épi-(tétrazolyl)-rapamycine (ABT578), 42-*O*-(2-hydroxy)éthyl-rapamycine (RAD001), AP23573, TAFA-93, et biolimus.

16. Inhibiteur de la revendication 1 pour utilisation selon la revendication 1, le mammifère à risque ayant un gène suppresseur de tumeur compromis, le gène suppresseur de tumeur étant *p53* ou *p16^{ink4a}.*
